(19) Europäisches Patentamt
European Patent Office
Office européen des brevets

(11) **EP 3 773 845 B1**

(12) **EUROPEAN PATENT SPECIFICATION**

(45) Date of publication and mention
of the grant of the patent:
**03.07.2024   Bulletin 2024/27**

(21) Application number: **19785042.3**

(22) Date of filing: **12.04.2019**

(51) International Patent Classification (IPC):
***A61M 16/06*** *(2006.01)*

(52) Cooperative Patent Classification (CPC):
**A61M 16/0683;** A61M 16/0633; A61M 16/1075;
A61M 16/16; A61M 2205/0216; A61M 2207/00

(86) International application number:
**PCT/NZ2019/050037**

(87) International publication number:
**WO 2019/199180 (17.10.2019 Gazette 2019/42)**

(54) **HEADGEAR FOR USE WITH A PATIENT INTERFACE OF A RESPIRATORY THERAPY SYSTEM**

KOPFBEDECKUNG ZUR VERWENDUNG MIT EINER PATIENTENSCHNITTSTELLE EINES
ATEMTHERAPIESYSTEMS

HARNAIS DESTINÉ À ÊTRE UTILISÉ AVEC UNE INTERFACE PATIENT D'UN SYSTÈME DE
THÉRAPIE RESPIRATOIRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **13.04.2018   US 201862657427 P**

(43) Date of publication of application:
**17.02.2021   Bulletin 2021/07**

(60) Divisional application:
**24165464.9**

(73) Proprietor: **Fisher & Paykel Healthcare Limited
East Tamaki
Auckland 2013 (NZ)**

(72) Inventor: **DUTHIE, Neil Gray
Auckland, 2013 (NZ)**

(74) Representative: **Dehns
St. Bride's House
10 Salisbury Square
London EC4Y 8JD (GB)**

(56) References cited:
EP-A2- 0 350 322          EP-A2- 2 022 528
WO-A1-2004/041341   WO-A1-2006/072128
WO-A1-2007/006089   WO-A1-2012/140514
WO-A1-2014/165906   WO-A1-2015/151019
WO-A1-2015/151019   WO-A1-2017/021836
WO-A1-2017/021836   WO-A1-2018/065926
WO-A1-2018/065926   WO-A9-2014/165906

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

## BACKGROUND OF THE DISCLOSURE

### Field of the Disclosure

The present disclosure generally relates to headgear for use with a patient interface of a respiratory therapy system.

### Description of the Related Art

One example of use of a respiratory therapy system is for the treatment of obstructive sleep apnea (OSA) by continuous positive airway pressure (CPAP) flow generator systems involving the continuous delivery of pressurized gases to the air-ways of a human via a breathing gas delivery conduit and a patient interface. Such a patient interface may be any one of the following:

- a nasal interface configured to seal around the nares or nose of the patient;

- an oral mask configured to seal around the mouth of the patient; or

- a full-face mask configured to seal around both the mouth and nose of the patient.

Headgear is typically used to secure the patient interface on the user's head. The headgear typically comprises one or more straps and/or panels that are secured to the patient interface and which pass around one or more parts of the patient's head. The headgear is typically configured to provide one or more of following functions:

a) to locate the patient interface in the desired position on the user's face.

b) to maintain the seal between the patient interface and the user's face by application of compression forces between the seal of the patient interface and the user's face.

c) to distribute the required forces between the patient interface and the user.

d) to improve the comfort of the patient during therapy.

e) to improve the appearance of the product. Whilst the appearance might be considered insubstantive, it has been shown that the aesthetics of components of a respiratory therapy system can affect how willing the user is to use the system and thus ultimately affect the success of the therapy.

f) to be cost effective to manufacture, and in particular to minimise material usage. This can be a particular concern for products that are disposable, or at least which are used for a relatively short period, such as less than one month for example. One example of this is in a hospital setting where patients are typically prescribed non-invasive ventilation (NIV) for relatively short periods of time in comparison to home treatments in which the patient may use the mask continuously for a longer period, such as up to six months, before replacing it. CPAP headgear may be washed and reused whereas typically NIV headgear is single use and is then disposed of.

Such headgear can comprise a number of different sizes and configurations of straps that are integrally formed, or joined together. Further achieving one or more of the above functions can be difficult, as one function can conflict with another.

An example of known headgear is that provided with the Simplus ® or Nivairo ® product of Fisher & Paykel Healthcare Limited. That particular product uses headgear comprising a pair of upper and lower side straps which are configured to be removably secured to respective parts of the patient interface. The upper and lower side straps extend along the sides of the user's head and terminate at a rear panel that engages the rear of the patient's head. A crown strap is further provided, which extends over the crown of the user's head from the upper side straps.

WO 2006/072128 A1 discloses a headgear assembly for attachment to a patient interface that delivers breathable gas to a patient. The headgear assembly includes a pair of side portions and a rear portion that interconnects the pair of side portions, each of the side portions including an upper side strap and a lower side strap, the rear portion including an upper strap, a lower strap and intermediate connecting straps extending between the upper strap and the lower strap.

WO2018065926 discloses a headgear which can include at least two upper side straps, at least two lower side straps, a rear panel, and/or a crown strap, among other components. The upper side straps can be positioned on opposite sides of the headgear and can extend downwardly from the crown strap at an angle which allows the upper side straps to extend downwardly above the user's ears to the mask. The lower side straps can be positioned on opposite sides of the headgear. In use, the lower side straps can extend substantially horizontally below the ears of the user. The lower side straps can extend from the rear panel to towards the mask.

## SUMMARY OF THE DISCLOSURE

The present disclosure stems from work undertaken towards providing improved headgear to better fulfil any one or more of the above functions.

It is an object of the present disclosure to provide an improved headgear for use with a patient interface, and/or that will at least provide the public or the medical profession with a useful choice.

The present invention provides headgear configured to be secured to a patient interface of a respiratory therapy system, to mount the patient interface on a user's head, the headgear comprising; two pairs of side straps, including an upper pair of side straps and a lower pair of side straps, each side strap of each pair having first and second opposed ends, a first end of each strap being secured to, or being configured to be secured to, the patient interface and to extend along the sides of the user's head; a rear panel configured to engage the rear of the user's head, the second end of each side strap being joined to the rear panel such that each pair of side straps extend from the rear panel; wherein the rear panel comprises a main body and a pair of laterally extending arms, the second end of each side strap of the upper pair being joined to a respective arm of the rear panel and connected entirely to the respective arm of the rear panel in an upper corner region of the rear panel, the second end of each side strap of the lower pair being connected to a lower corner area of the rear panel, the lower corner area including at least a portion of the respective arm and the main body, wherein the upper side straps and the lower side straps are inclined towards each other at their first ends; the main body comprising: opposed top and base margins which are spaced apart by a distance, opposed side margins extending between the top and base margins, and a central longitudinal axis bisecting the top and base margins; each laterally extending arm being inclined upwardly away from the main body and away from the central longitudinal axis, extending outwardly from the central longitudinal axis away from a respective side margin of the main body and comprising a distal arm end, each distal arm end having a width measured in a direction substantially parallel with the central longitudinal axis; wherein the width of each distal arm end is between 30% to 60% of the distance between the opposed top and base margins of the main body.

The side margins of the main body of the rear panel may be at least partially substantially parallel and substantially equal length such that the main body is substantially rectangular.

The side margins of the main body of the rear panel may be of substantially equal length and may be at least partially inclined relative to the base margin such that the main body is substantially trapezoidal.

Each lateral arm may be of substantially uniform width along its length.

Each distal arm end may comprise a substantially straight end margin. The end margin may be parallel with the longitudinal axis. The end margin may be inclined relative to the longitudinal axis. The end margin may be inclined relative to the longitudinal axis at an angle between 0.1 and 20°.

The side margins of the rear panel may be inclined relative to the longitudinal axis at a different angle to the angle of inclination of the lateral arms relative to the longitudinal axis.

The side margins of the rear panel may be inclined at an angle between 0° and 40° relative to the longitudinal axis of the rear panel.

The longitudinal axis of each arm may be inclined at an angle between 40° and 90° relative to the longitudinal axis of the rear panel.

The entire rear panel may be constrained within a notional rectangle of 100-300mm wide by 50-120, tall, and in some embodiments within a notional rectangle of 180-200mm by 75-95mm.

The rear panel may be defined by any one or more of the following ratios:

a. the ratio of the length of a lateral arm to the length of the main portion is between 0.65 and 0.8, length being measured in a direction perpendicular to the longitudinal axis;

b. the ratio of the height of a lateral arm to the height of the main portion, height being measured in a direction parallel to the longitudinal axis, is between 1.1 and 1.6;

c. the ratio of the length of the entire rear panel to the length of the main portion is between 2.0 and 3.0;

d. the ratio of the area of a lateral arm to the area of the main portion is between 0.3 and 0.5;

e. the ratio of the area of both lateral arms to the area of the entire rear panel is between 0.3 and 0.7.

f. the ratio of a cut out or recess formed adjacent the upper margin of the rear panel as defined by the area between the upper margin of the rear panel and a notional horizontal line extending between and connecting the two most upper points of the lateral arms, to the area of the entire rear panel is between 0.3 and 0.5.

g. the rear panel comprises between 30 and 70% of a notional rectangular area that surrounds and contacts all extreme points of the rear panel.

The rear panel may be defined by any one or more of the following ratios:

a. the total area of the rear panel is between 8000 and 13000mm$^2$;
b. the ratio of the area of both lateral arms to the area of the main portion of the rear panel is between 0.1 and 0.3.
c. the ratio of the area of the main portion to the entire area of the main panel is between 0.75 and 1.0.

The rear panel may be defined by the following formula:

$$w2 = 1/2\, d1 - 1/2\, c;$$

where w2 = the width of the lateral arm as measured in a direction substantially parallel with the longitudinal axis, d1 = the distance between opposed top and base margins of the main portion, and c = the clearance between adjacent rear panels required during manufacture of multiple rear panels on a sheet of material to ensure a satisfactory quality of cut.

Each inclined arm may extend from a respective side margin and the top margin of the main body.

The main body of the rear panel may comprise opposing top and base margins, opposing side margins connecting the opposing top and base margins, a central vertical plane connecting the mid points of the opposing top and base margins, and a central horizontal plane connecting the mid points of the opposing side margins,

wherein the opposing side margins are inclined relative to the opposing top and base margin, and are of equal length, such that the main body is substantially trapezoidal;
wherein each inclined arm extends from one of the pair of side margins and the top margin of the trapezoidal shaped main body above the central horizontal plane and extend away from both the central horizontal and vertical planes, and at an angle between 0° and 90° from the horizontal plane.

Each angled arm may extend away from one of the pair of side margins and the top margin of the trapezoidal shaped main body, and the area of each angled arm is less than 15% of the area of the main body.

Each angled arm may extend from one of the pair of side margins and the top margin of the main body, and each upper side strap connects entirely to a respective one of the pair of angled arms and the at least one of the pair of lower straps connects entirely to the main body.

Each laterally extending arm may taper, flare or remain substantially constant along its length.

The distal arm end of each laterally extending arm may be stepped or tapered or waisted, so that the distal arm end is of narrower width than the remainder of the laterally extending arm.

The ratio of the width of each laterally extending arm to the width of the main body portion may be between 0.4:1 and 0.8:1.

Also described herein is headgear configured to be secured to a patient interface of a respiratory therapy system, to mount the patient interface on a user's head, the headgear comprising;

a rear panel configured to engage the rear of a user's head,
a top strap extending from the rear panel and configured engage the crown of the user's head,
a pair of upper side straps extending from the top strap, and
a pair of lower side straps extending from the rear panel,
the upper and lower side straps being secured to, or being configured to be secured to, the patient interface;
the rear panel comprising a main body and a pair of inclined arms extending outwardly from the main body, wherein the main body comprises opposing top and base margins which are spaced apart by a distance, and opposing side margins connecting the opposing top and base margins, wherein
the opposing side margins are inclined relative to the opposing top and base margin, and are of equal length, such that the main body is substantially trapezoidal;
each inclined arm extending from a respective side margin and the top margin of the main body;
the angled arms of the rear panel having a width which is between 30% to 60% the distance between the base and top margins of the main body.

Also described herein is headgear configured to be secured to a patient interface of a respiratory therapy system, to mount the patient interface on a user's head, the headgear comprising;

a rear panel configured to engage the rear of a user's head;
a pair of upper side straps extending from the rear panel, and
a pair of lower side straps extending from the rear panel,
the upper and lower side straps being secured to, or being configured to be secured, to the patient interface;
the rear panel comprising a main body and a pair of opposed, laterally extending inclined arms, the main body comprising opposing top and base margins, opposing side margins connecting the opposing top and base margins, a central vertical plane connecting the mid points of the opposing top and base margins, and a central horizontal plane connecting the mid points of the opposing side margins,
wherein the opposing side margins are inclined relative to the opposing top and base margins, and are of substantially equal length, such that the main body is substantially trapezoidal;
wherein each inclined arm extends from a respective one of the pair of side margins and the top margin of the trapezoidal shaped main body, each inclined arm extending from a position above the central horizontal plane and away from both the central horizontal and vertical planes, and at an angle between 0° and 90° from the horizontal plane.

Also described herein is headgear configured to be secured to a patient interface of a respiratory therapy system, to mount the patient interface on a user's head, the headgear comprising:

a rear panel configured to engage the rear of a user's head,
a pair of upper side straps extending from the rear panel, and
a pair of lower side straps extending from the rear panel,
the upper and lower side straps being secured to, or being configured to be secured to, the patient interface;
the rear panel comprising a main body and a pair of opposed, laterally extending inclined arms, the main body comprising opposing top and bottom margins and opposing side margins connecting the opposing top and bottom margins,
wherein the opposing side margins are inclined relative to the opposing top and base margin, and are of substantially equal length, such that the main body is substantially trapezoidal;
wherein each angled arm extends away from a respective one of the pair of side margins and the top margin of the trapezoidal shaped main body,
wherein the area of each angled arm is less than 15% of the area of the main body.

Also described herein is headgear configured to be secured to a patient interface of a respiratory therapy system, to mount the patient interface on a user's head, the headgear comprising:

a rear panel configured to engage the rear of a user's head,
a pair of upper side straps extending from the rear panel, and
a pair of lower side straps extending from the rear panel,
the upper and lower side straps being secured to, or being configured to be secured to, the patient interface;
the rear panel comprising a main body and a pair of opposed, laterally extending inclined arms, the main body comprising opposing top and bottom margins and opposing side margins connecting the opposing top and bottom margins,
wherein the opposing side margins are inclined relative to the opposing top and base margin, and are of substantially equal length, such that the main body is substantially trapezoidal;
each angled arm extends from a respective one of the pair of side margins and the top margin of the main body,
wherein each upper side strap connects entirely to a respective one of the pair of angled arms, and each lower side strap connects entirely to the main body.

The invention is defined by the appended claims. Further aspects of the disclosure, which should be considered in all its novel aspects, will become apparent from the following description.

## DESCRIPTION OF THE DRAWINGS

A number of embodiments of the disclosure will now be described by way of example with reference to the drawings in which:

**Figure 1** is a perspective view from the rear of a headgear in accordance with the present disclosure;
**Figure 2** is a perspective view from the rear and side of the headgear of Figure 1;
**Figure 3** is a rear view of the headgear of Figures 1 and 2;

**Figure 4** is a plan view of the exterior of the headgear of Figures 1 to 3 when laid flat;

**Figure 5** is a plan view of the interior of the headgear of Figures 1 to 4 when laid flat;

**Figures 6 to 9** are plan views of a rear panel of the headgear of Figures 1 to 5, showing different dimensions and aspects of the rear panel;

**Figure 10** is a lay flat view of a continuous sheet of textile in an industry standard width of 59" (1498mm) with an example of the nesting patterns and yield that can be achieved with the rear panel of Figures 6 to 9;

**Figure 11** is a hypothetical nesting pattern of two rear panels of headgear in accordance with the current disclosure;

**Figure 12** is an enlarged view corresponding to Figure 10;

**Figures 13 to 15** are lay flat views of a continuous sheet of textile in an industry standard width of 59" (1498mm) with an example of other nesting patterns and yield that can be achieved with the rear panel of headgear in accordance with the current disclosure;

**Figures 16 to 19** are plan views of a rear panel of headgear in accordance with the current disclosure showing different dimensions and aspects of the rear panel;

**Figures 20 to 25** are plan views of another embodiment of a rear panel of the headgear of Figures 1 to 5, showing different dimensions and aspects of the rear panel with Figure 22 being an enlarged view of Area A of Figure 24; and

**Figures 26 to 31** are plan views of a further embodiment of a rear panel of the headgear of Figures 1 to 5, showing different dimensions and aspects of the rear panel, with Figure 28 being an enlarged view of Area A of Figure 29j.

## DETAILED DESCRIPTION

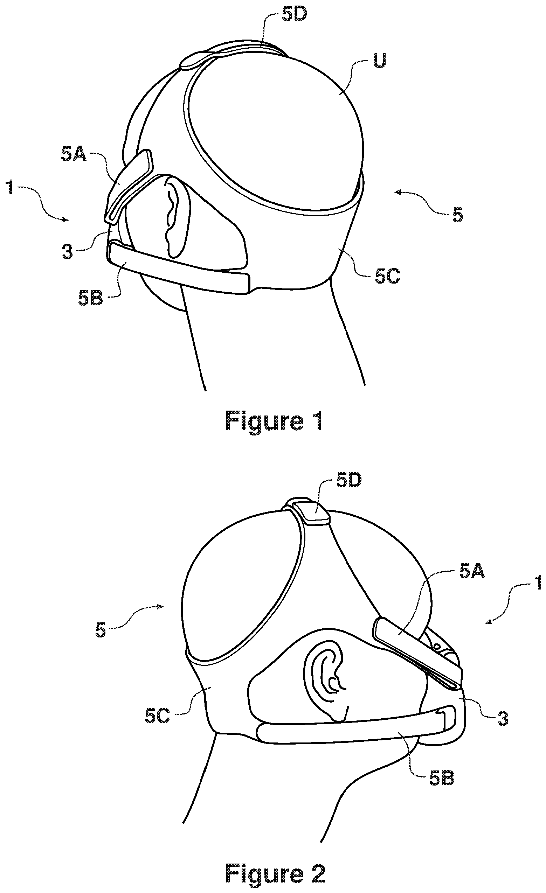

With reference initially to Figures 1 to 3, an embodiment of a patient interface assembly 1 comprising a patient interface 3 and headgear 5 is illustrated on a user U, the patient interface in this example being a full face mask covering both the nose and mouth of the user U. The patient interface assembly 1 can be used in the field of respiratory therapy and therefore in any respiratory treatment, respiratory assistance, resuscitation or ventilation system. In some embodiments, the interface assembly 1 has particular utility with forms of positive pressure respiratory therapy. For example, the interface assembly 1 can be used for administering continuous positive airway pressure ("CPAP") treatments, variable positive airway pressure ("VPAP") treatments and/or bi-level positive airway pressure ("BiPAP") treatments. The interface assembly 1 can be compatible with one or more different types of suitable CPAP or non-invasive ventilation (NIV) systems.

The patient interface 3 can comprise any of a plurality of different types of suitable mask configurations. For example, certain features, aspects and advantages of the present disclosure can be utilized with nasal masks, full face masks, oronasal masks, total face, or any other positive pressure mask. Although the illustrated mask is a full face mask, the scope of the present disclosure should not be limited by the particular embodiments described.

In the illustrated configuration, the patient interface 3 comprises a mask body, optionally a mask frame and a connection port assembly. The mask body is configured to cover the user's mouth and/or nose to deliver respiratory gases to the user. The mask body can be secured to the mask frame 5. The patient interface 3 is held in place on the user U by the headgear 5 that wraps around a part or parts of the user's head. The connection port assembly can be connected to the mask body and/or mask frame, preferably with a releasable connection. In some configurations, the connection port assembly comprises an elbow connector configured to be connected between the mask body and/or mask frame and a gas delivery conduit (not shown).

The mask frame can couple to the mask body and help stabilize the interface 3 on the user's face. The mask frame can be any shape and size to functionally secure the interface 3 to the user's face. The mask frame can be attached to the mask body with interlocking clips, tabs or other functional couplers or may be permanently attached during the assembly process. In this latter case the mask body and mask frame are integral and configured to form a single component. The mask frame can be rigid, substantially rigid or semi-rigid to provide support for the mask body. For example, the mask frame can be at least partially made of a metal or rigid plastic, such as acrylic, polycarbonate or high-density polyethylene.

The mask frame can extend to the user's forehead and may optionally include a forehead rest. The forehead rest, if provided, can help stabilize the interface 1 to the user's face by providing a support point for the interface 1 and connection points for the headgear 5.

If provided, the forehead rest can be a separate flexible piece that is attached or overmoulded onto the mask frame. For example, the forehead rest can be made of a flexible silicone that is overmoulded onto the frame bridge. The flexible material advantageously conforms to the user's forehead anatomy and helps improve comfort to the user with soft material contact. In some configurations, the forehead rest can be attached or integrally formed as part of the mask frame and can be made of the same material as the mask frame 5 and frame bridge.

In this example headgear 5 comprises a pair of upper side straps 5A extending from laterally spaced upper connections on the mask body or mask frame, a pair of lower side straps 5B extending from laterally spaced lower connections on the mask body or mask frame, a rear panel 5C which joins the upper and lower side straps 5A, 5B, at the rear of the head of the user U, and a crown strap 5D which extends over the crown of the head of the user U, from each upper side strap 5A. Suitable connectors, such as incorporating hook and loop fasteners, may be provided to secure the headgear 5 to the mask body or mask frame. In this example, both pairs of upper and lower straps 5A, 5B comprise hook and loop fasteners 6 at their respective strap ends, each strap being looped through the mask frame and/or forehead rest and back on itself. The amount by which each strap 5A, 5B is looped back on itself can provide some adjustment of the size and fit of the headgear 5. Both pairs of upper and lower straps 5A, 5B are further provided with gripping formations 8 at their distal ends.

Any or all of the above straps 5A. 5B, 5C may include adjusters, such as buckles, configured to enable the length of the strap(s) to be adjusted.

Any one or more of the straps 5A. 5B, 5C and/or rear panel 5C may be elastic or inelastic or comprise portions that are elastic or inelastic. Any one or more the straps 5A. 5B, 5C or rear panel 5C may comprise more rigid portions, for example, to help maintain a desired shape of the headgear 5. The materials, construction, and elasticity may vary between straps 5A. 5B, 5C and/or rear panel 5C however, the headgear 5 alternatively may be of uniform construction with the same material and elasticity used throughout the headgear. This may further reduce costs by enabling the entire assembly to be cut from sheets of the same material.

In this example crown strap 5C comprises two crown strap portions 5DA, 5DB that are releasably connected together via the end of one crown strap portion 5DA being received in an aperture 21 of the other crown strap portion 5DB. The end of first crown strap portion 5DA is waisted such that there is a narrower width portion 23 spaced from the end of the first crown strap portion 5DA. The waisted portion 23 is substantially the same width as the aperture 21 and is located in the aperture 21 when the two crown strap portions 5DA, 5DB are connected together, with the wider end 24 of first crown strap portion 5DA protruding through the aperture and resisting removal of the first crown strap portion 5DA through the aperture 21.

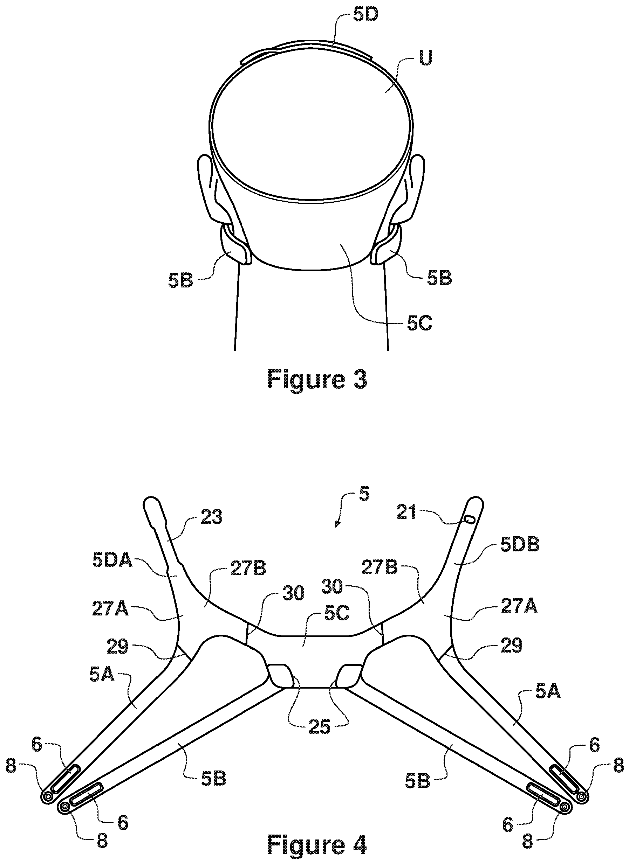

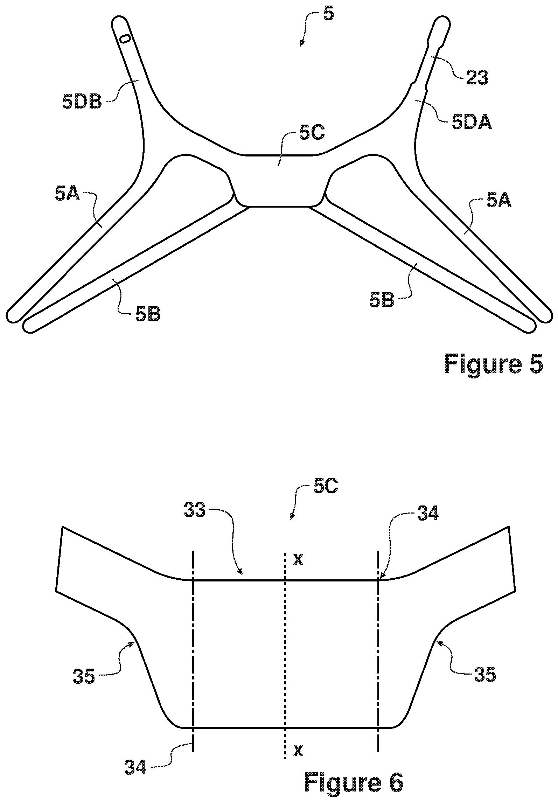

With reference additionally to Figures 4 and 5, the margins of the rear panel 5C are shown, with the lower side straps 5B being joined to the rear panel 5C at joins 25. The inner end of each crown strap portion 5DA, 5DB comprises a bifurcated region 27 forming two legs 27A, 27B. The upper side straps 5A are joined to respective legs 27A at joins 29 whilst legs 27B are joined 30 to respective upper margins 31 of the rear panel 5C. The joins may use any suitable joining means which can include any one or more of stitching, gluing, ultra-sonic welding, RF welding or any other form of seam welding.

The upper and lower side straps 5A, 5B are joined to the rear panel 5C such that the upper straps 5A are non-parallel with the lower side straps 5B such that the distal ends of the upper and lower side straps 5A, 5B on each side of the rear panel 5C are inclined toward one another. The angle between the upper and lower side straps 5A, 5B can be varied depending on the connection of the upper and lower side straps 5A, 5B to the patient interface.

Referring additionally to Figure 6, the rear panel 5C comprises a main portion or body 33 and two opposed lateral portions or arms 35, with a notional transition line 34 between the main and lateral portions 33, 35. In this example, the main portion 33 can be considered to be rectangular. The two lateral portions 35 each extend outwardly from the central plane or longitudinal axis XX and are inclined upwardly away from the main portion 33 and away from the longitudinal axis to facilitate connection with the upper straps 5A and locate those straps 5A in the correct position on the back of the user's head.

Each upper strap 5A is connected entirely to a respective one of the lateral portions 35 of the rear panel 5C in an upper corner region of the rear panel 5C. Each lower strap 5B is connected to a lower corner area of the rear panel 5C, that includes at least a portion of the lateral portion 35 and main portion 33.

The boundaries of the main portion 33 are defined by a centrally located rectangle with upper and lower limits formed by the substantially straight parallel regions of the upper and lower margins of the rear panel 5C and lateral limits formed by notional vertical lines 34 the ends of which are located at points where the upper margin transitions from substantially horizontal by a line of best fit, to a curved line into the lateral portions 35. By 'substantially horizontal', we include that the upper margin is slightly curved.

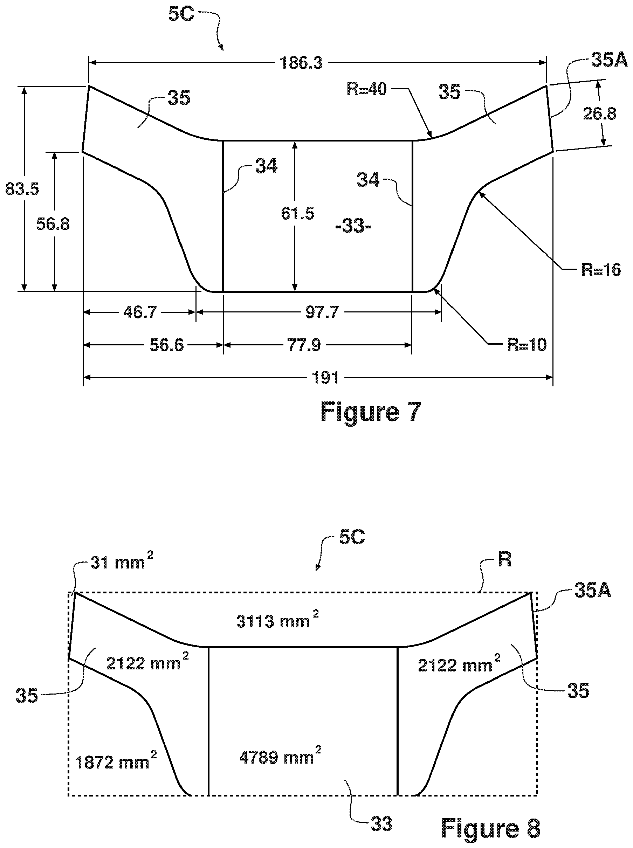

Referring to Figure 7, example relative dimensions (mm) of the rear panel 5C are:

- The main portion 33 is defined by a rectangle with dimensions of less than 100mm by 70mm, and in one example 77.9mm by 61.5mm.
- The lateral portions 35 are constrained within a rectangle of less than 60mm by 90mm, and in one example 56.6mm by 83.5 mm.
- The entire rear panel 5C is constrained within a rectangle R, shown in dashed line, of less than 200mm by 100mm, and in one example 191mm by 83.5mm.
- The ratio of the area of each lateral portion 35 to the area of the main portion 33 is between 0.8 and 0.9 and preferably 0.886.

Referring to Figure 8 the rear panel 5C can be effectively described with various example ratios relating the lateral portions 35 and main portion 33. Example ratios are listed below.

- The ratio of the width of a lateral portion 35 to the width of the main portion 33 may be between 0.7 and 0.8, and in one example is 0.73.
- The ratio of the height of a lateral portion 35 to the height of the main portion 33 may be between 1.2 and 1.5, and in one example is 1.36.
- The ratio of the width of the entire rear panel 5C to the width of the main portion 33 may be between 2.0 and 3.0, and in one example is 2.45.
- The ratio of the area of a lateral portion 35 to the area of the main portion 33 is may be between 0.4 and 0.5, and in one example 0.443.
- The ratio of the area of both lateral portions 35 to the area of the entire rear panel 5C may be between 0.4 and 0.7, and in one example is 0.470.
- The ratio of a cutout formed in the top of the rear panel 5C defined by the area A between the upper margin 41 of the rear panel 5C and a horizontal line 43 connecting the two most upper points 45 of the lateral portions 35 to the area of the entire rear panel 5C may be between 0.3 and 0.5, and in one example is 0.347.
- The rear panel 5C may be between 30 and 70%, and in one example makes up 56.64%, of a notional rectangular area A that extends to touch all extreme points of the rear panel 5C.
- The ratio of the width of the lower margin of the rear panel 5C to the width of the entire rear panel 5C may be between 0.4 and 0.8, and in one example is 0.51.

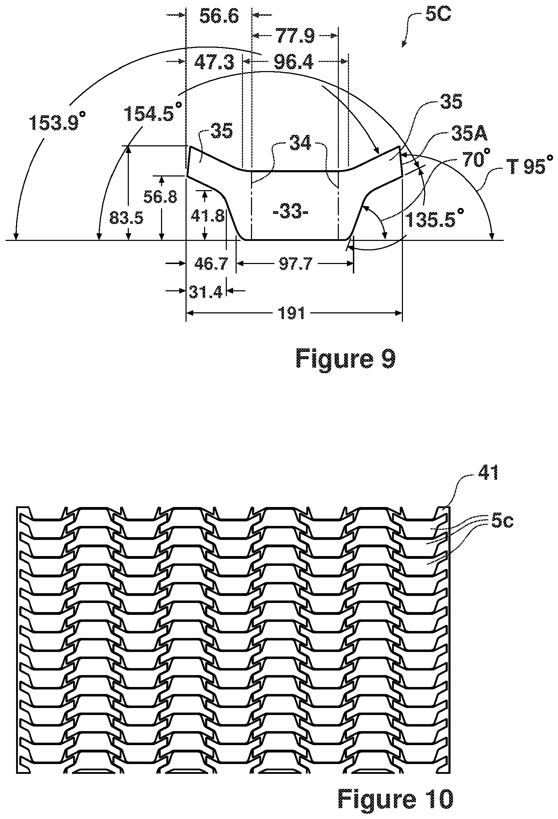

Referring to Figure 9, example dimensions of, and example angles between, the different parts of the rear panel 5C are shown. These include a lateral portion 35 tip angle T which defines the angle of the straight, distal end margin 35A of each lateral portion 35 relative to the horizontal base margin of the main portion 33.

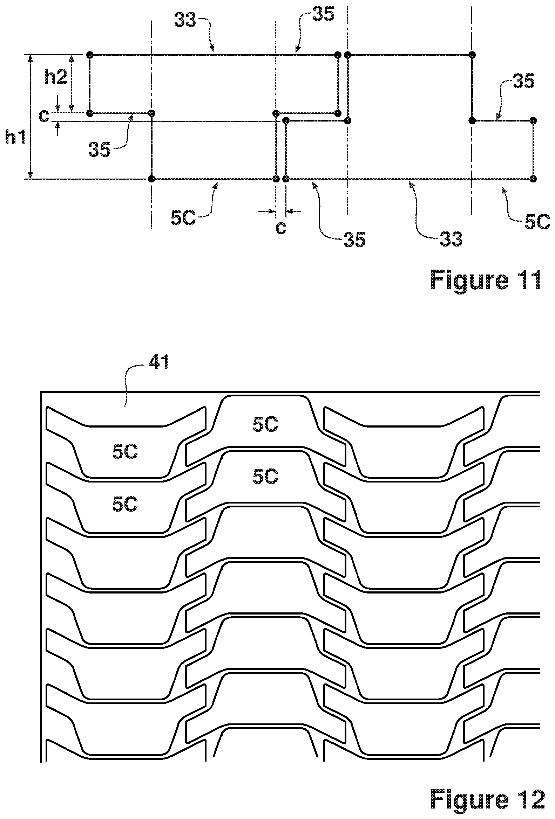

Referring to Figures 10 and 12, a continuous sheet of textile 41 in an industry standard width of 59" (1498mm) is shown with an example of the nesting patterns and yield that can be achieved with a rear panel 5C in accordance with this disclosure. It is desirable to firstly use an industry standard width of material, and to secondly maximise the amount of that material that is used, minimising the amount that is wasted and therefore reducing the overall manufacturing cost of the headgear assembly.

- the shape and configuration of the rear panel 5C is such that a relatively high yield is possible when adjacent panels 5C are placed alongside each other with each intermediate panel 5C in a given row of rear panels 5C being inverted relative to the adjacent panel 5C.
- In this position the angles and shape of the lateral portions 35 are complimentary with each other which allows the lateral portions of each row to be positioned between the lateral portions of the adjacent rows without the rear panels contacting each other. This arrangement allows for a relatively high yield nesting pattern which decreases material waste and reduces the cost of the component due to the disused areas formed between rows of rear panels being inhabited partially/substantially by the adjacent rows of rear panels.
- In this disclosure yields of over 75% are possible, and in this example a yield of 83.9% can be achieved.

Referring to Figure 11, a hypothetical nesting pattern of two rear panels 5D that could achieve the maximum yield possible while leaving a clearance c of 3.175mm (1/8th of an inch) between pieces to enable the die cutting blades to effectively operate is shown. This nesting pattern uses the following formula:

$$w2 = 1/2\ h1\ -\ 1/2\ c;$$

where w2 = the width of the lateral arm as measured in a direction substantially parallel with the longitudinal axis, d1 = the distance between opposed upper and base margins of the main portion, and c = the clearance between adjacent rear panels required during manufacture of multiple rear panels on a sheet of material to ensure a satisfactory quality of cut.

The clearance c can be varied in order to achieve a satisfactory cut between adjacent rear panels 5D. It is desirable from a material wastage point of view that this clearance is as small as possible. However, if the clearance equals zero, the quality of the cut margin of each rear panel 5 may be compromised.

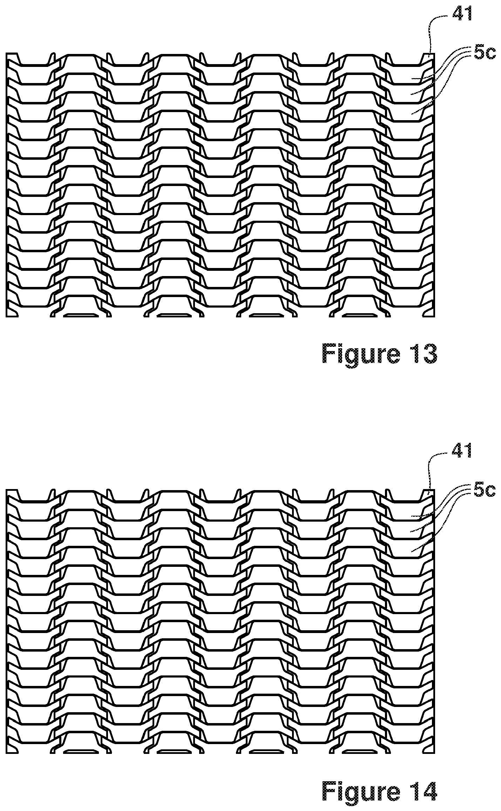

Figures 13 to 15 show different examples of the rear panel 5C where the tip angle T of the of the straight end margin 35B of each lateral portion 35 relative to the horizontal base margin 33A of the main portion 33 is varied, this tip angle T affecting the yield. In each of these examples a yield of over 83% is achieved. Although the tip angle T will affect the available yield, this angle may also affect the aesthetics of the headgear, and/or the best angle to achieve a suitable joint with the upper side strap 5A. As such choosing the best tip angle T may require consideration of any one or more of these factors, and/or some compromise.

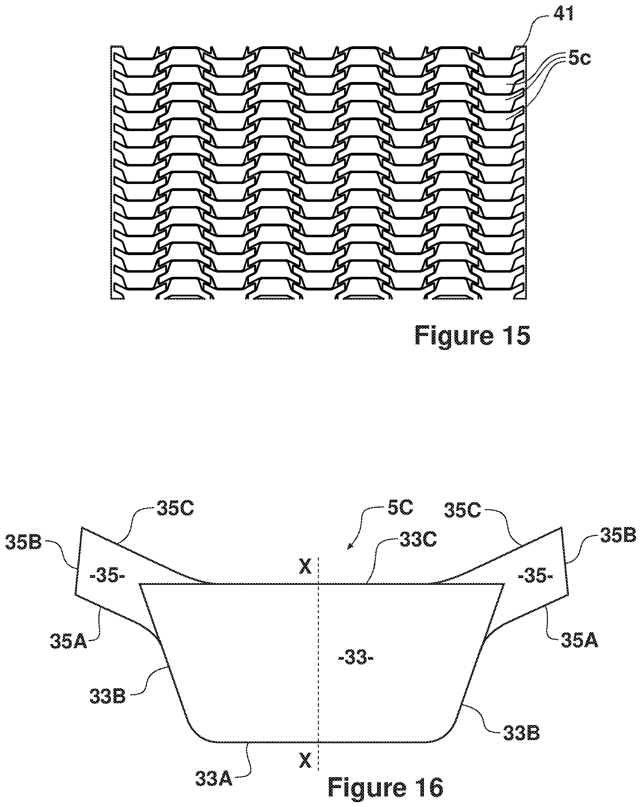

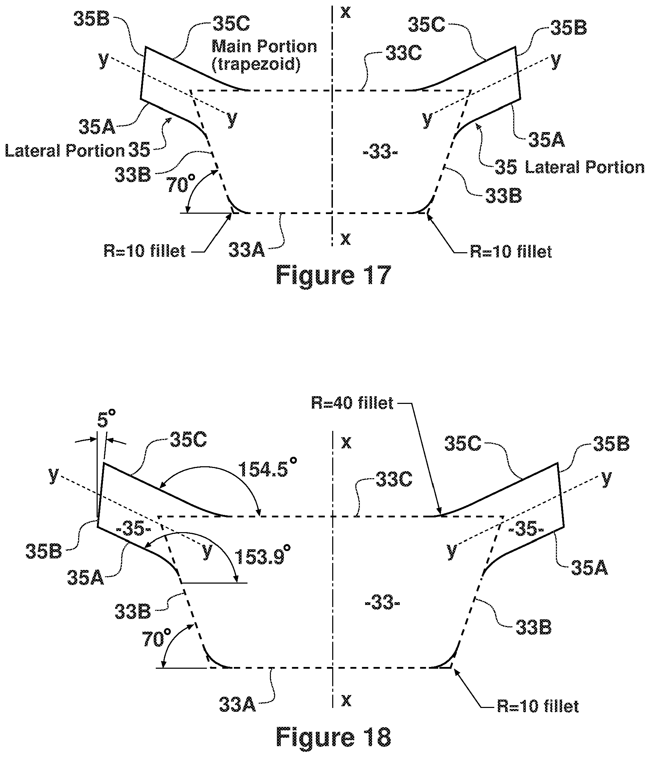

Referring to Figures 16 to 18, the rear panel 5C is considered in an alternative manner as comprising a trapezoidal main portion 33 with two lateral portions 35 each intersecting the trapezoidal main portion 33.

Each lateral portion 35 extends laterally outwardly, and is inclined, away from main portion 33 and from the central vertical plane/longitudinal axis XX such that a longitudinal axis YY of each lateral portion 35 is inclined relative to the longitudinal axis XX and relative to the straight, horizontal upper and lower margins of the main portion 33.

The rear panel 5C may therefore be defined by a main portion or body 33 comprising a substantially straight horizontal lower base margin 33A leading to substantially straight, outwardly inclined side margins 33B. Each side margin 33B leads to a respective lateral portion 35 comprising an elongate outwardly extending arm where the longitudinal axis YY of each arm 35 is inclined relative to the base margin 33A, and the angle of inclination of each arm 35 relative to the base margin 33A is less than the angle of inclination of the side margins 33B. Each lateral arm 35 comprises substantially straight lower, end and upper margins 35A, 35B, 35C, with the end margin 35B being inclined relative to the central longitudinal axis XX of the rear panel 5C. The lower and upper margins 35A, 35C could be parallel, or could taper toward or away from another. The upper margin of each arm 35 leads into the substantially straight upper margin 33C of the panel 5C, the upper margin 33C being parallel with the base margin 33A. The lower margin 35A of each arm 35 leads into a respective side margin 33B of the main body 33.

When considered in this way:

- The rear panel 5C is symmetrical about line XX.
- The straight, horizontal upper and lower margins 33C, 33A of the main portion 33 are parallel.
- The outwardly inclined side margins 33B of the main portion 33, each extend from a separate lateral endpoint of base margin 33A at an angle of 70°, such that side margins 33B extend upwardly and outwardly from the central plane XX.
- The endpoints of side margins 33B intersect the endpoints of top margin 33C such that base margin 33A, side margins 33B and top margin 33C together form a trapezoidal shape which is mirrored about plane XX with top margin 33C > base margin 33A > side margins 33B.
- The intersections of side margins 33B with base margin 33A are filleted with a 10mm radius.
- The two lateral portions 35 extend distally away from the central plane XX at an angle above horizontal.
- Lower arm margin 35A intersects side margin 33B and extends distally away from the central plane XX at an angle of between 15 and 45°, and in one example of 26.1° from a plane parallel to base margin 33A.
- The intersection between lower arm margin 35A and base margin 33A is filleted with a 16mm radius.
- Upper line margin 35C intersects top margin 33C and extends distally away from the central plane XX at an angle of between 15 and 45°, and in one example of 25.5°, from top margin 33C.
- The intersection between upper arm margin 35C and top margin 33C is filleted, in this example with a 40mm radius.
- End arm margin 35B extends from the free end of lower arm margin 35A at an angle of between 0° and 20°, and in this example 5°, from a plane parallel to plane XX such that is extends proximally towards plane XX and intersects the free end of upper arm margin 35C.

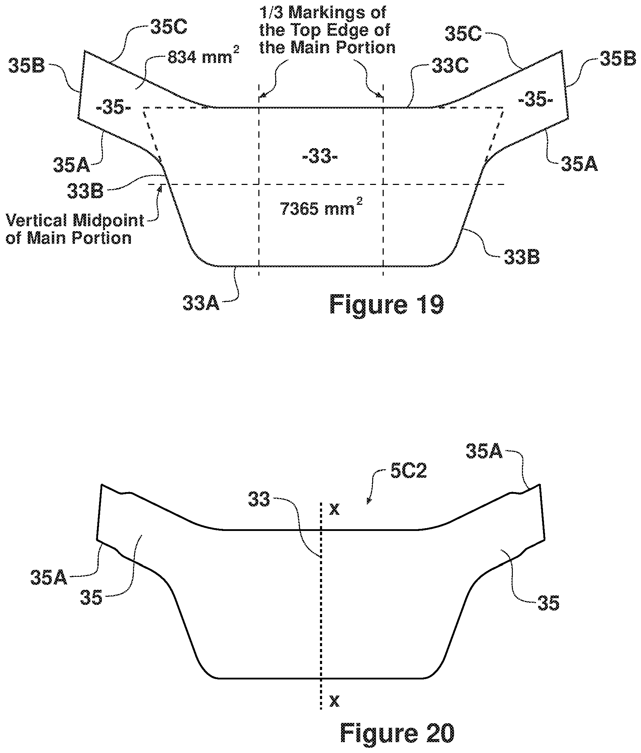

As illustrated in Figure 19:

- The total area of the rear panel 33 may be between 6000 and 13000mm$^2$, and in this example is about 9,000mm$^2$.
- The ratio of the area of both lateral portions 35 to the main portion 33 may be between 0.1 and 0.3, and in this example is about 0.185.
- The ratio of the area of the main portion 33 to the total area of the rear panel 5C may be between 0.7 and 1.0, and in this example is about 0.815.
- The lateral portions 35 intersect the main portion 33 above the vertical midpoint of the main portion.
- With the length of the top edge margin 33C of the main portion 33 divided into two lateral thirds and a central third, each lateral portion 35 can be said to intersect the top edge margin 33C of the main portion 33 within the lateral third proximal to the lateral portion 35 in question.

In this disclosure the main portion or body 33 is defined by the area captured within the top and base margins 33C, 33A and the side margins 33B, including the area defined where the top margin 33C notionally extends towards the lateral portions or arms 35 and intersects with where the side margins 33B notionally extend towards to the lateral portions or arms 35. These notionally extending parts of the top and side margins 33C, 33B are shown in dotted line N in Figure 19 for example.

The headgear 5 may be configured such that the other dimensions and ratios set out above can vary in dependence upon the size of head gear 5 (and rear panel 5A) required. In other words, the rear panel 5A may be configured to be scaled such that the proportions and ratios of various components will remain the same between the smallest to largest size of the rear panel 5A. The panel 5A is configured to be scaleable such that for example the major dimensions of a large size may be 1.5 times bigger than the major dimensions of a small size.

Table 1 below shows example dimensions of three headgear sizes, for headgear 5 in accordance with this disclosure. The headgear rear panel 5C described above is for a size Medium/Large. This is designed to fit a majority of a given set of users. The other two size options show examples of a largest practical rear panel size as well as a smallest practical rear panel size. However, the particular example dimensions listed in the table below are designed to be able to fit the largest and smallest head sizes of which we are aware, and illustrate that variations in proportions between different sizes is possible, i.e. this table lists possible extreme dimensions and as such these three size configurations are not scaled relative to one another as outlined above.

| | Medium / L Rear Panel Dimensions (Adult Patient) | Largest Practical Rear Panel Dimensions (Adult Patient) | Smallest Practical Rear Panel Dimensions (Adult Patient) |
| --- | --- | --- | --- |
| Overall Rear Panel Width | 191mm | 261mm | 121mm |
| Central Quadrilateral Region Width | 77.9mm | 150mm | 10mm |
| Overall Rear Panel Height | 83.5mm | 118.5mm | 50mm |

The headgear 5 may provide a relatively low cost solution that is important for use in the NIV environment where masks and associated headgear are disposable and each patient receives a new mask every 14 days typically. The relatively low cost solution is achieved via a unique geometrical shape and configuration that allows relatively high yield manufacturing owing to the efficient nesting pattern. The relatively large single component rear panel 5C may also increase patient comfort and/or improve the aesthetics of the headgear 5, which may help to increase patient compliance.

The headgear 5 may comprise part of a respiratory therapy system which comprises any one or more of:

a. a breathing gas flow generator;

b. a breathing gas humidifier;

c. a gas delivery conduit, which may or may not be heated;

d. a patient interface.

The headgear 5 may comprise part of a patient interface assembly comprising the combination of the headgear 5 and a patient interface 3.

The headgear 5 may be manufactured from any suitable fabric material or combination of materials. Parts of the headgear 5 may for example be manufactured from Breathoprene ®.

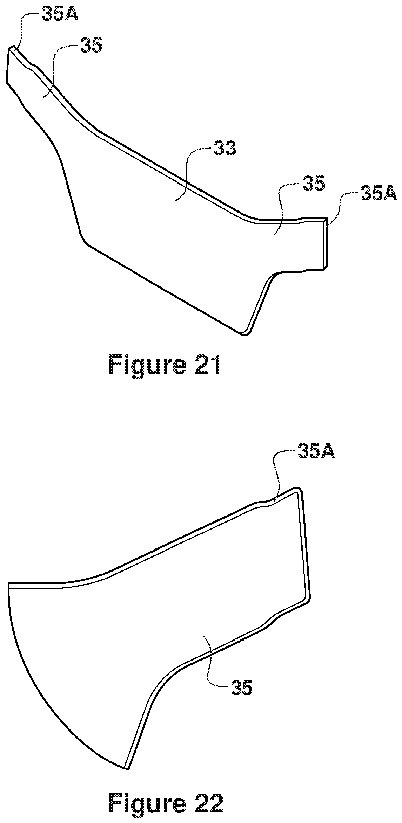

Referring to Figures 20 to 25, another embodiment of rear panel 5C is shown, and will be referred to as rear panel 5C2. Panel 5C2 is closely similar to Panel 5C described above, and like features will be given like references. Only the differences are discussed below.

Rear panel 5C2 can again be considered to comprise a main portion or body 33 and two opposed lateral portions or arms 35, with a notional transition line 37 between the main and lateral portions 33, 35. In this example, the main portion 33 can be considered to be rectangular. The two lateral portions 35 each extend outwardly from the central plane or longitudinal axis XX and are inclined upwardly away from the main portion 33 and away from the longitudinal axis to facilitate connection with the upper straps 5A and locate those straps 5A in the correct position on the back of the user's head. The lateral portions 35 of panel 5C2 terminate in tips 35A of reduced width. These are configured to minimise laminate flare when welded to straps of the headgear.

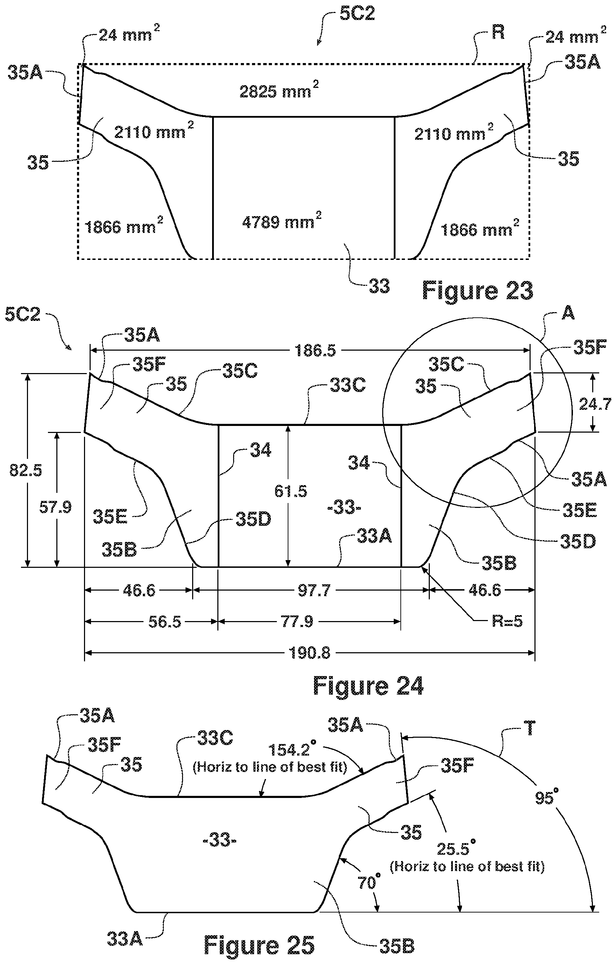

Referring to Figure 24, the example relative dimensions (mm) of the rear panel 5C2 are identical or closely similar to that of panel 5C except that the tip of each lateral arm 35 is approximately 2mm narrower, for the reason discussed above. The radius of curvature between the lower margin of main body 33 and each lateral arm 35 is about 5mm in this example, as compared to about 10mm for panel 5C.

Referring to Figure 24 the rear panel 5C2 can be effectively described with the same example ratios relating the lateral portions 35 and main portion 33, as described above for panel 5C. Example ratios are listed below.

Referring to Figure 25, example dimensions of, and example angles between, the different parts of the rear panel 5C2 are shown. These are substantially the same as those of panel 5C.

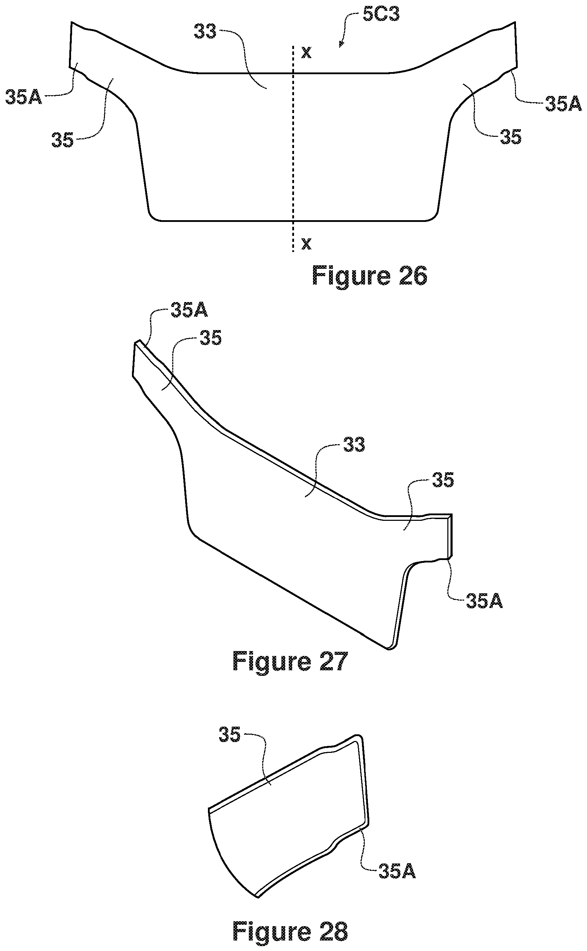

Referring to Figures 26 to 31, another embodiment of rear panel 5C is shown, and will be referred to as rear panel SC3. Panel 5C3 is closely similar to panels 5C and SC2 described at length above, and like features will be given like references. Panel SC3 is a very similar height to panels 5C and SC2, but is narrower overall, with a wider main portion 33, narrower width lateral portions 35, and lateral portions 35 that are more steeply inclined relative to the horizontal (of the base margin of main portion 33).

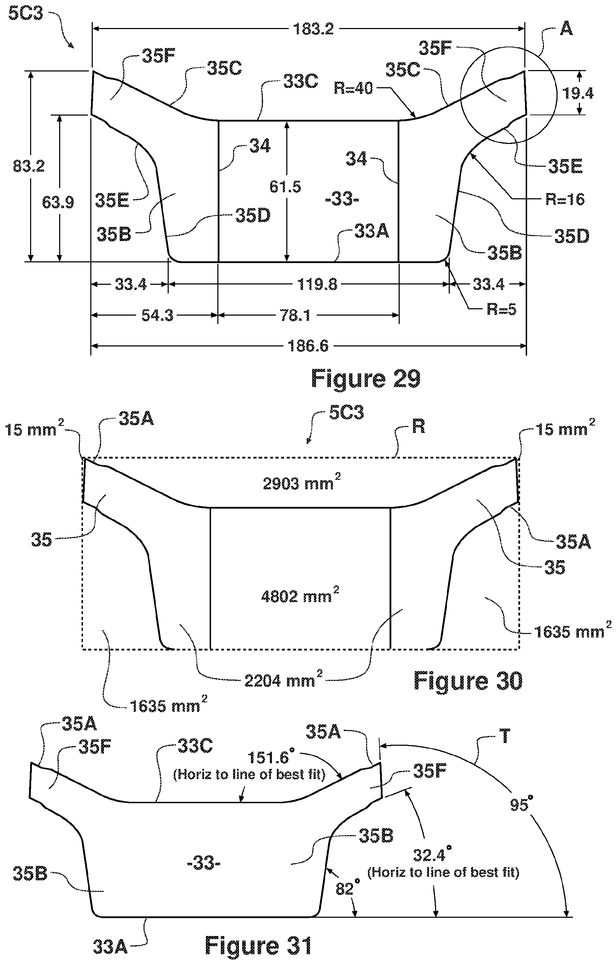

Referring to Figure 29, example relative dimensions (mm) of the rear panel 5C3 are:

- The main portion 33 is defined by a rectangle with dimensions of less than 100mm by 70mm, and in one example 78.1mm by 63.9mm.
- The lateral portions 35 are constrained within a rectangle of less than 60mm by 90mm, and in one example 54.3mm by 83.2 mm.
- The entire rear panel 5C is constrained within a rectangle R, shown in dashed line, of less than 200mm by 100mm, and in one example 186.6mm by 83.2mm.
- The ratio of the area of the lateral portions 35 to the area of the main portion 33 is between 0.8 and 0.9 and preferably 0.92.

As with rear panels 5C and 5C2, each lateral portion 35 can be considered to comprise a stem portion 35B which extends from main body portion 33 and is defined between the main body portion 33 and a lower side margin comprising lower and upper parts 35D, 35E. Each lateral portion 35 further comprises a projecting portion 35F which projects radially outwardly from the stem portion 35B and is defined by the upper part 35E of lower side margin, the tip 35A, and an upper margin 35C. Each of margins 35C, 35D, 35E are substantially straight in these examples, although one or more of them could be arcuate. The upper part 35D of lower side margin and the upper margin 35C may be substantially parallel, or they may be inclined toward or away from one another, towards tip 35A. Thus the projecting portion 35F may taper or flare along its length, or be of substantially constant width.

In respect of panel 5C3, the stem portion 35B is wider than that of panels 5C, 5C2, and is approximately 20% wider in this example. The lower part of each lateral arm 35 is therefore more oblong than panels 5C, 5C2, and inclines upwardly away from the base margin of main portion 33 at a steeper angle such that the stem part of each lateral arm 35 initially does not project outwardly as far as arms 35 of panels 5C, 5C2. For panels 5C, 5C2, the ratio of the width of each lateral arm 35 to the width of the main body portion 33 is between 0.4:1 and 0.5:1. For panel 5C3, the ratio of the width of each lateral arm 35 to the width of the main body portion 33 is between 0.6:1 and 0.8:1. These calculations are based on each lateral arm 35 terminating where the upper arm margin 35F joins the straight, horizontal part of the upper margin 33C of main body portion 33. This termination point is defined by a vertical plane 34 which extends through the intersection of upper arm margin 35C with the upper margin 33C of main body portion 33, as can best be seen in figures 9, 24 and 29.

Referring to Figure 30 the rear panel 5C3 can be effectively described with various example ratios relating the lateral portions 35 and main portion 33. Example ratios are listed below.

- The ratio of the width of a lateral portion 35 to the width of the main portion 33 may be between 0.7 and 0.8, and in one example is 0.69.
- The ratio of the height of a lateral portion 35 to the height of the main portion 33 may be between 1.2 and 1.5, and in one example is 1.30.
- The ratio of the width of the entire rear panel 5C3 to the width of the main portion 33 may be between 2.0 and 3.0, and in one example is 2.39.
- The ratio of the area of a lateral portion 35 to the area of the main portion 33 is may be between 0.4 and 0.5, and in one example is 0.46.
- The ratio of the area of both lateral portions 35 to the area of the entire rear panel 5C may be between 0.3 and 0.7, and in one example is 0.48.
- The ratio of a cut-out formed in the top of the rear panel 5C3 defined by the area A between the upper margin 41 of the rear panel 5C3 and a horizontal line 43 connecting the two most upper points 45 of the lateral portions 35 to the area of the entire rear panel 5C3 may be between 0.3 and 0.5, and in one example is 0.32.
- The rear panel 5C3 may be between 30 and 70%, and in one example makes up 59.32%, of a notional rectangular area A that extends to touch all extreme points of the rear panel 5C.
- The ratio of the width of the lower margin of the rear panel 5C3 to the width of the entire rear panel 5C3 may be between 0.4 and 0.8, and in one example is 0.64.

Referring to Figure 31, example dimensions of, and example angles between, the different parts of the rear panel 5C3 are shown. These include a lateral portion 35 tip angle T which defines the angle of the straight, distal end margin 35A of each lateral portion 35 relative to the horizontal base margin 33A of the main portion 33.

The angle between the lower part of the lower side margin of each lateral portion 35 and the base margin 33A of main portion 33 may be between 50 and 90°, for panel 5C and 5C2 is 70°, and for panel 5C3 is 82°. The lower part of the lower side margin is the part which extends from the base margin 33A of main portion 33. The angle between the upper part 35D of lower side margin (and the longitudinal axis of the projecting arm portion) of each lateral portion 35 and the base margin 33A of main portion 33 may be between 25 and 70°, for panel 5C is 45.5°, and for panel 5C3 is 32.4°.

The angle between the upper side margin 35D of each lateral portion 35 and the base margin 33A of main portion 33 may be between 100 and 180°, for panel 5C and 5C2 is 154.5°, and for panel 5C3 is 151.6°. The upper side margin 35D is the part which extends from the top margin 33C of main portion 33 to the tip 35A of each lateral portion 35.

The end margin of the tip 35A of each lateral portion 35 is preferably substantially straight. The angle between the end margin of the tip 35A of each lateral portion 35, and the base margin 33A of main portion 33 is preferably between 80 and 120°, and for panels 5C, 5C2 and 5C3 is 95°.

Unless the context clearly requires otherwise, throughout the description, the words "comprise", "comprising", and the like, are to be construed in an inclusive sense as opposed to an exclusive or exhaustive sense, that is to say, in the sense of "including, but not limited to".

Any discussion of the prior art throughout the specification should in no way be considered as an admission that such prior art is widely known or forms part of common general knowledge in the field.

## Claims

1. Headgear (5) configured to be secured to a patient interface (3) of a respiratory therapy system, to mount the patient interface on a user's head, the headgear comprising;

   two pairs of side straps (5A, 5B), including an upper pair of side straps (5A) and a lower pair of side straps (5B), each side strap of each pair having first and second opposed ends, a first end of each strap being secured to, or being configured to be secured to, the patient interface (3) and to extend along the sides of the user's head; a rear panel (5C) configured to engage the rear of the user's head, the second end of each side strap being joined to the rear panel such that each pair of side straps extend from the rear panel (5C); wherein the rear panel (5C) comprises a main body (33) and a pair of laterally extending arms (35), the second end of each side strap of the upper pair (5A) being joined to a respective arm (35) of the rear panel (5C) and connected entirely to the respective arm (35) of the rear panel (5C) in an upper corner region of the rear panel (5C), the second end of each side strap of the lower pair (5B) being connected to a lower corner area of the rear panel (5C), the lower corner area including at least a portion of the respective arm (35) and the main body (33), wherein the upper side straps and the lower side straps are inclined towards each other at their first ends; the main body (33) comprising:

   opposed top and base margins which are spaced apart by a distance, opposed side margins extending between the top and base margins, and a central longitudinal axis bisecting the top and base margins;

   each laterally extending arm (35) being inclined upwardly away from the main body (33) and away from the central longitudinal axis, extending outwardly from the central longitudinal axis away from a respective side margin of the main body (33) and comprising a distal arm end, each distal arm end having a width measured in a direction substantially parallel with the central longitudinal axis; **characterised in that** the width of each distal arm end is between 30% to 60% of the distance between the opposed top and base margins of the main body (33).

2. The headgear of claim 1, wherein the side margins of the main body (33) of the rear panel (5C) are substantially parallel and substantially equal length such that the main body (33) is substantially rectangular.

3. The headgear of claim 1, wherein the side margins of the main body (33) of the rear panel (5C) are substantially equal length and are inclined relative to the base margin such that the main body (33) is substantially trapezoidal.

4. The headgear of any one of the preceding claims wherein each lateral arm (35) is of substantially uniform width along its length.

5. The headgear of any one of the preceding claims wherein the distal arm end comprises a substantially straight end margin.

6. The headgear of claim 5 wherein the end margin is parallel with the longitudinal axis.

7. The headgear or claim 5 wherein the end margin is inclined relative to the longitudinal axis, optionally inclined at an angle between 0.1 and 20°.

8. The headgear of claim 3 wherein the side margins of the rear panel (5C) are inclined relative to the longitudinal axis at a different angle to the angle of inclination of the lateral arms (35) relative to the longitudinal axis.

9. The headgear of claim 3 wherein the side margins of the rear panel (5C) are inclined at an angle between 0° and 40° relative to the longitudinal axis of the rear panel (5C).

10. The headgear of any one of the preceding claims wherein the longitudinal axis of each arm (35) is inclined at an angle between 40° and 90° relative to the longitudinal axis of the rear panel (5C).

11. The headgear of any one of the preceding claims when dependent on claim 2 wherein the rear panel (5C) can be defined by any one or more of the following ratios:

   a. the ratio of the length of a lateral arm to the length of the main body (33) is between 0.65 and 0.8, length being measured in a direction perpendicular to the longitudinal axis;
   b. the ratio of the height of a lateral arm (35) to the height of the main body (33), height being measured in a direction parallel to the longitudinal axis, is between 1.1 and 1.6;
   c. the ratio of the length of the entire rear panel (5C) to the length of the main body (33) is between 2.0 and 3.0;
   d. the ratio of the area of a lateral arm (35) to the area of the main body (33) is between 0.3 and 0.5;
   e. the ratio of the area of both lateral arms (35) to the area of the entire rear panel (5C) is between 0.3 and 0.7.
   f. the ratio of a cut out or recess formed adjacent the upper margin of the rear panel (5C) as defined by the area between the upper margin of the rear panel and a notional horizontal line extending between and connecting the two most upper points of the lateral arms, to the area of the entire rear panel is between 0.3 and 0.5.
   g. the rear panel (5C) comprises between 30 and 70% of a notional rectangular area that surrounds and contacts all extreme points of the rear panel (5C).

12. The headgear of any one of the preceding claims when dependent on claim 3 wherein the rear panel (5C) can be defined by any one or more of the following:

   a. the total area of the rear panel (5C) is between 8000 and 13000mm$^2$;
   b. the ratio of the area of both lateral arms (35) to the area of the main body (33) of the rear panel is between 0.1 and 0.3.
   c. the ratio of the area of the main body (33) to the entire area of the rear panel (5C) is between 0.75 and 1.0.

13. The headgear of any one of the preceding claims where the rear panel (5C) is defined by the following formula;
   w2=1/2 d1 - 1/2 c;
   where w2 = the width of the lateral arm (35) as measured in a direction substantially parallel with the longitudinal axis, d1 = the distance between opposed top and base margins of the main body (33), and c = the clearance between adjacent rear panels required during manufacture of multiple rear panels on a sheet of material to ensure a satisfactory quality of cut.

14. The headgear of claim 3 wherein the main body (33) of the rear panel (5C) comprises opposing top and base margins, opposing side margins connecting the opposing top and base margins, a central vertical plane connecting the mid points of the opposing top and base margins, and a central horizontal plane connecting the mid points of the opposing side margins,

   wherein the opposing side margins are inclined relative to the opposing top and base margin, and are of equal length, such that the main body (33) is substantially trapezoidal;
   wherein each inclined arm (35) extends from one of the pair of side margins and the top margin of the trapezoidal shaped main body (33) above the central horizontal plane and extend away from both the central horizontal and vertical planes, and at an angle between 0° and 90° from the horizontal plane.

**Patentansprüche**

1.  Kopfbedeckung (5), die so konfiguriert ist, dass sie an einer Patientenschnittstelle (3) eines Atemtherapiesystems gesichert werden kann, um die Patientenschnittstelle am Kopf eines Benutzers anzubringen, wobei die Kopfbedeckung umfasst:

    zwei Paar Seitengurte (5A, 5B), die ein oberes Paar Seitengurte (5A) und ein unteres Paar Seitengurte (5B) beinhalten, wobei jeder Seitengurt jedes Paares erste und zweite gegenüberliegende Enden aufweist, wobei ein erstes Ende jedes Gurtes an der Patientenschnittstelle (3) gesichert ist oder so konfiguriert ist, dass es an dieser gesichert werden kann, und sich entlang der Seiten des Kopfes des Benutzers erstreckt;
    eine hintere Platte (5C), die so konfiguriert ist, dass sie die Hinterseite des Kopfes des Benutzers in Eingriff nimmt, wobei das zweite Ende jedes Seitengurts so mit der hinteren Platte zusammengefügt ist, dass sich jedes Paar Seitengurte von der hinteren Platte (5C) erstreckt; wobei
    die hintere Platte (5C) einen Hauptkörper (33) und ein Paar sich seitlich erstreckender Arme (35) umfasst, wobei das zweite Ende jedes Seitengurts des oberen Paares (5A) mit einem jeweiligen Arm (35) der hinteren Platte (5C) zusammengefügt und in einem oberen Eckbereich der hinteren Platte (5C) vollständig mit dem jeweiligen Arm (35) der hinteren Platte (5C) verbunden ist, wobei das zweite Ende jedes Seitengurts des unteren Paares (5B) mit einem unteren Eckbereich der hinteren Platte (5C) verbunden ist, wobei der untere Eckbereich mindestens einen Abschnitt des jeweiligen Arms (35) und des Hauptkörpers (33) beinhaltet, wobei die oberen Seitengurte und die unteren Seitengurte an ihren ersten Enden zu einander hin geneigt sind;
    wobei der Hauptkörper (33) umfasst:

    einen Oberrand und gegenüberliegenden Basisrand, die um einen Abstand voneinander beabstandet sind, gegenüberliegende Seitenränder, die sich zwischen dem Oberrand und Basisrand erstrecken, und eine Mittellängsachse, die den Oberrand und Basisrand halbiert;

    wobei jeder sich seitlich erstreckende Arm (35) vom Hauptkörper (33) weg und von der Mittellängsachse weg nach oben geneigt ist, sich von der Mittellängsachse von einem jeweiligen Seitenrand des Hauptkörpers (33) weg nach außen erstreckt und ein distales Armende umfasst,
    wobei jedes distale Armende eine in einer im Wesentlichen zur Mittellängsachse parallelen Richtung gemessene Breite aufweist;
    **dadurch gekennzeichnet, dass**
    die Breite jedes distalen Armendes zwischen 30 % bis 60 % des Abstands zwischen dem Oberrand und gegenüberliegenden Basisrand des Hauptkörpers (33) beträgt.

2.  Kopfbedeckung nach Anspruch 1, wobei die Seitenränder des Hauptkörpers (33) der hinteren Platte (5C) im Wesentlichen parallel und im Wesentlichen gleich lang sind, sodass der Hauptkörper (33) im Wesentlichen rechteckig ist.

3.  Kopfbedeckung nach Anspruch 1, wobei die Seitenränder des Hauptkörpers (33) der hinteren Platte (5C) im Wesentlichen gleich lang sind und relativ zum Basisrand so geneigt sind, dass der Hauptkörper (33) im Wesentlichen trapezförmig ist.

4.  Kopfbedeckung nach einem der vorstehenden Ansprüche, wobei jeder seitliche Arm (35) entlang seiner Länge von im Wesentlichen einheitlicher Breite ist.

5.  Kopfbedeckung nach einem der vorstehenden Ansprüche, wobei das distale Armende einen im Wesentlichen geraden Endrand umfasst.

6.  Kopfbedeckung nach Anspruch 5, wobei der Endrand zur Längsachse parallel ist.

7.  Kopfbedeckung nach Anspruch 5, wobei der Endrand relativ zur Längsachse geneigt ist, gegebenenfalls in einem Winkel zwischen 0,1 und 20° geneigt ist.

8.  Kopfbedeckung nach Anspruch 3, wobei die Seitenränder der hinteren Platte (5C) relativ zur Längsachse in einem Winkel geneigt sind, der sich vom Neigungswinkel der seitlichen Arme (35) relativ zur Längsachse unterscheidet.

9.  Kopfbedeckung nach Anspruch 3, wobei die Seitenränder der hinteren Platte (5C) in einem Winkel zwischen 0° und 40° relativ zur Längsachse der hinteren Platte (5C) geneigt sind.

**10.** Kopfbedeckung nach einem der vorstehenden Ansprüche, wobei die Längsachse jedes Arms (35) in einem Winkel zwischen 40° und 90° relativ zur Längsachse der hinteren Platte (5C) geneigt ist.

**11.** Kopfbedeckung nach einem der vorstehenden Ansprüche in Abhängigkeit von Anspruch 2, wobei die hintere Platte (5C) durch eines oder mehrere der folgenden Verhältnisse definiert sein kann:

a) das Verhältnis der Länge eines seitlichen Arms zur Länge des Hauptkörpers (33) liegt zwischen 0,65 und 0,8, wobei die Länge in einer Richtung senkrecht zur Längsachse gemessen wird;
b) das Verhältnis der Höhe eines seitlichen Arms (35) zur Höhe des Hauptkörpers (33) liegt zwischen 1,1 und 1,6, wobei die Höhe in einer Richtung parallel zur Längsachse gemessen wird;
c) das Verhältnis der Länge der gesamten hinteren Platte (5C) zur Länge des Hauptkörpers (33) liegt zwischen 2,0 und 3,0;
d) das Verhältnis der Fläche eines seitlichen Arms (35) zur Fläche des Hauptkörpers (33) liegt zwischen 0,3 und 0,5;
e) das Verhältnis der Fläche beider seitlichen Arme (35) zur Fläche der gesamten hinteren Platte (5C) liegt zwischen 0,3 und 0,7,
f) das Verhältnis eines Ausschnitts oder einer Aussparung, die an den oberen Rand der hinteren Platte (5C) angrenzend gebildet ist, wie durch die Fläche zwischen dem oberen Rand der hinteren Platte und einer gedachten horizontalen Linie, die sich zwischen den zwei obersten Punkten der seitlichen Arme erstreckt und diese verbindet, definiert, zur Fläche der gesamten hinteren Platte liegt zwischen 0,3 und 0,5,
g) die hintere Platte (5C) umfasst zwischen 30 und 70 % einer gedachten rechteckigen Fläche, die alle Extrempunkte der hinteren Platte (5C) umgibt und berührt.

**12.** Kopfbedeckung nach einem der vorstehenden Ansprüche in Abhängigkeit von Anspruch 3, wobei die hintere Platte (5C) durch eines oder mehrere der Folgenden definiert sein kann:

a) die Gesamtfläche der hinteren Platte (5C) beträgt zwischen 8000 und 13000 mm$^2$;
b) das Verhältnis der Fläche beider seitlicher Arme (35) zur Fläche des Hauptkörpers (33) der hinteren Platte liegt zwischen 0,1 und 0,3,
c) das Verhältnis der Fläche des Hauptkörpers (33) zur gesamten Fläche der hinteren Platte (5C) liegt zwischen 0,75 und 1,0.

**13.** Kopfbedeckung nach einem der vorstehenden Ansprüche, wobei die hintere Platte (5C) durch die folgende Formel definiert ist; w2=1/2 d1 - 1/2 C;
wobei w2 = die Breite des seitlichen Arms (35), in einer im Wesentlichen zur Längsachse parallelen Richtung gemessen, d1 = der Abstand zwischen dem Oberrand und gegenüberliegenden Basisrand des Hauptkörpers (33), und c = der Freiraum zwischen aneinandergrenzenden hinteren Platten, der bei der Herstellung mehrerer hinterer Platten auf einer Materialbahn erforderlich ist, um eine zufriedenstellende Schnittqualität sicherzustellen.

**14.** Kopfbedeckung nach Anspruch 3, wobei der Hauptkörper (33) der hinteren Platte (5C) einen Oberrand und gegenüberliegenden Basisrand, gegenüberliegende Seitenränder, die den Oberrand und gegenüberliegenden Basisrand verbinden, eine mittlere vertikale Ebene, die die Mittelpunkte des Oberrands und gegenüberliegenden Basisrands verbindet, und eine mittlere horizontale Ebene, die die Mittelpunkte der gegenüberliegenden Seitenränder verbindet, umfasst,

wobei die gegenüberliegenden Seitenränder relativ zum gegenüberliegenden Ober- und Basisrand geneigt sind und von gleicher Länge sind, sodass der Hauptkörper (33) im Wesentlichen trapezförmig ist;
wobei sich jeder geneigte Arm (35) von einem des Paares Seitenränder und dem Oberrand des trapezförmig geformten Hauptkörpers (33) über die mittlere horizontale Ebene erstreckt und sowohl von der mittleren horizontalen als auch der vertikalen Ebene weg und in einem Winkel zwischen 0° und 90° von der horizontalen Ebene erstreckt.

**Revendications**

**1.** Harnais (5) configuré pour être fixé à une interface patient (3) d'un système de thérapie respiratoire, pour monter l'interface patient sur la tête d'un utilisateur, le harnais comprenant ;

deux paires de sangles latérales (5A, 5B), incluant une paire supérieure de sangles latérales (5A) et une paire inférieure de sangles latérales (5B), chaque sangle latérale de chaque paire présentant des première et seconde extrémités opposées, une première extrémité de chaque sangle étant fixée à, ou étant configurée pour être fixée à, l'interface patient (3) et pour s'étendre le long des côtés de la tête de l'utilisateur ;

un panneau arrière (5C) configuré pour venir en prise avec l'arrière de la tête de l'utilisateur, la seconde extrémité de chaque sangle latérale étant reliée au panneau arrière de telle sorte que chaque paire de sangles latérales s'étend depuis le panneau arrière (5C) ; dans lequel

le panneau arrière (5C) comprend un corps principal (33) et une paire de bras s'étendant latéralement (35), la seconde extrémité de chaque sangle latérale de la paire supérieure (5A) étant reliée à un bras respectif (35) du panneau arrière (5C) et entièrement relié au bras respectif (35) du panneau arrière (5C) dans une région de coin supérieur du panneau arrière (5C), la seconde extrémité de chaque sangle latérale de la paire inférieure (5B) étant reliée à une zone de coin inférieure du panneau arrière (5C), la zone de coin inférieure incluant au moins une partie du bras respectif (35) et du corps principal (33), dans lequel les sangles latérales supérieures et les sangles latérales inférieures sont inclinées les unes vers les autres à leurs premières extrémités ;

le corps principal (33) comprenant :

des bordures supérieure et de base opposées qui sont espacées d'une certaine distance,
des bordures latérales opposées s'étendant entre les bordures supérieure et de base, et
un axe longitudinal central coupant en deux les bordures supérieure et de base ;

chaque bras s'étendant latéralement (35) étant incliné vers le haut en s'éloignant du corps principal (33) et en s'éloignant de l'axe longitudinal central, s'étendant vers l'extérieur depuis l'axe longitudinal central en s'éloignant d'une bordure latérale respective du corps principal (33) et comprenant une extrémité distale de bras, chaque extrémité distale de bras présentant une largeur mesurée dans une direction sensiblement parallèle à l'axe longitudinal central ;

**caractérisé en ce que**
la largeur de chaque extrémité distale de bras est comprise entre 30 % et 60 % de la distance entre les bordures supérieure et de base opposées du corps principal (33).

2. Harnais selon la revendication 1, dans lequel les bordures latérales du corps principal (33) du panneau arrière (5C) sont sensiblement parallèles et de longueur sensiblement égale de telle sorte que le corps principal (33) soit sensiblement rectangulaire.

3. Harnais selon la revendication 1, dans lequel les bordures latérales du corps principal (33) du panneau arrière (5C) sont sensiblement de même longueur et sont inclinées par rapport à la bordure de base de telle sorte que le corps principal (33) soit sensiblement trapézoïdal.

4. Harnais selon l'une quelconque des revendications précédentes, dans lequel chaque bras latéral (35) présente une largeur sensiblement uniforme sur sa longueur.

5. Harnais selon l'une quelconque des revendications précédentes, dans lequel l'extrémité distale de bras comprend une bordure d'extrémité sensiblement droite.

6. Harnais selon la revendication 5, dans lequel la bordure d'extrémité est parallèle à l'axe longitudinal.

7. Harnais selon la revendication 5, dans lequel la bordure d'extrémité est inclinée par rapport à l'axe longitudinal, éventuellement inclinée selon un angle compris entre 0,1 et 20°.

8. Harnais selon la revendication 3, dans lequel les bordures latérales du panneau arrière (5C) sont inclinées par rapport à l'axe longitudinal selon un angle différent de l'angle d'inclinaison des bras latéraux (35) par rapport à l'axe longitudinal.

9. Harnais selon la revendication 3, dans lequel les bordures latérales du panneau arrière (5C) sont inclinées selon un angle compris entre 0° et 40° par rapport à l'axe longitudinal du panneau arrière (5C).

10. Harnais selon l'une quelconque des revendications précédentes, dans lequel l'axe longitudinal de chaque bras (35) est incliné selon un angle compris entre 40° et 90° par rapport à l'axe longitudinal du panneau arrière (5C).

**11.** Harnais selon l'une quelconque des revendications précédentes lorsqu'elle dépend de la revendication 2, dans lequel le panneau arrière (5C) peut être défini par un ou plusieurs quelconques des rapports suivants :

a) le rapport entre la longueur d'un bras latéral et la longueur du corps principal (33) est compris entre 0,65 et 0,8, la longueur étant mesurée dans une direction perpendiculaire à l'axe longitudinal ;
b) le rapport entre la hauteur d'un bras latéral (35) et la hauteur du corps principal (33), la hauteur étant mesurée dans une direction parallèle à l'axe longitudinal, est compris entre 1,1 et 1,6 ;
c) le rapport entre la longueur de l'ensemble du panneau arrière (5C) et la longueur du corps principal (33) est compris entre 2,0 et 3,0 ;
d) le rapport entre la surface d'un bras latéral (35) et la surface du corps principal (33) est compris entre 0,3 et 0,5 ;
e) le rapport entre la surface des deux bras latéraux (35) et la surface de l'ensemble du panneau arrière (5C) est compris entre 0,3 et 0,7.
f) le rapport d'une découpe ou d'un évidement formé à côté de la bordure supérieure du panneau arrière (5C), tel que défini par la zone située entre la bordure supérieure du panneau arrière et une ligne horizontale fictive s'étendant entre et reliant les deux points les plus élevés des bras latéraux, à la surface de l'ensemble du panneau arrière est compris entre 0,3 et 0,5.
g) le panneau arrière (5C) comprend entre 30 et 70 % d'une zone rectangulaire fictive qui entoure et touche tous les points extrêmes du panneau arrière (5C).

**12.** Harnais selon l'une quelconque des revendications précédentes lorsqu'elle dépend de la revendication 3, dans lequel le panneau arrière (5C) peut être défini par un ou plusieurs quelconques des éléments suivants :

a) la surface totale du panneau arrière (5C) est comprise entre 8 000 et 13 000 mm$^2$;
b) le rapport entre la surface des deux bras latéraux (35) et la surface du corps principal (33) du panneau arrière est compris entre 0,1 et 0,3.
c) le rapport entre la surface du corps principal (33) et la surface totale du panneau arrière (5C) est compris entre 0,75 et 1,0.

**13.** Harnais selon l'une quelconque des revendications précédentes, dans lequel le panneau arrière (5C) est défini par la formule suivante : w2=1/2 d1 - 1/2 c ;
dans lequel w2 = la largeur du bras latéral (35), mesurée dans une direction sensiblement parallèle à l'axe longitudinal, d1 = la distance entre les bordures supérieure et de base opposées du corps principal (33), et $c$ = l'espace libre entre les panneaux arrière adjacents nécessaire lors de la fabrication de plusieurs panneaux arrière sur une feuille de matériau pour garantir une qualité de découpe satisfaisante.

**14.** Harnais selon la revendication 3, dans lequel le corps principal (33) du panneau arrière (5C) comprend des bordures supérieure et de base opposées, des bordures latérales opposées reliant les bordures supérieure et de base opposées, un plan vertical central reliant les points médians des bordures supérieure et de base opposées et un plan horizontal central reliant les points médians des bordures latérales opposées,

dans lequel les bordures latérales opposées sont inclinées par rapport aux bordures supérieure et de base opposées, et sont de longueur égale, de telle sorte que le corps principal (33) est sensiblement trapézoïdal ;
dans lequel chaque bras incliné (35) s'étend depuis l'une des deux bordures latérales et la bordure supérieure du corps principal de forme trapézoïdale (33) au-dessus du plan horizontal central et s'éloigne à la fois du plan horizontal central et du plan vertical central, et selon un angle entre 0° et 90° par rapport au plan horizontal.

Figure 1

Figure 2

Figure 3

Figure 4

5

23

5DB

5DA

5C

5A

5A

5B

5B

**Figure 5**

5C

33

34

X

35

35

34

X

**Figure 6**

**Figure 7**

**Figure 8**

Figure 9

Figure 10

Figure 11

Figure 12

41

5c

Figure 13

41

5c

Figure 14

Figure 15

Figure 16

35B      35C      x      35C      35B

y

Main Portion
(trapezoid)

33C

y

y

35A

Lateral Portion 35

33B

70°

y

-33-

35A

35 Lateral Portion

33B

R=10 fillet      33A      x      R=10 fillet

**Figure 17**

5°

35C

R=40 fillet

x

35C

35B

154.5°

y

33C

y

35B

-35-

y

153.9°

y

-35-

35A

35A

33B

-33-

70°

33B

33A      x      R=10 fillet

**Figure 18**

35C

1/3 Markings of
the Top Edge of
the Main Portion

35C

35B

834 mm$^2$

33C

35B

-35-

-35-

-33-

35A

35A

33B

7365 mm$^2$

33B

Vertical Midpoint
of Main Portion

33A

Figure 19

5C2

35A

33

X

35A

35

35

X

Figure 20

Figure 21

Figure 22

Figure 23

Figure 24

Figure 25

Figure 26

Figure 27

Figure 28

Figure 29

Figure 30

Figure 31

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2006072128 A1

- WO 2018065926 A